# EUROPEAN PATENT SPECIFICATION

(11) **EP 2 611 416 B1**
(45) Date of publication and mention of the grant of the patent: **19.08.2015**
(21) Application number: 11761534.4
(22) Date of filing: 05.09.2011
(51) Int. Cl.: A61K 9/00, A61M 15/00, A61K 9/14

(54) **PHARMACEUTICAL COMPOSITION SUITABLE FOR USE IN A DRY POWDER INHALER**
PHARMAZEUTISCHE ZUBEREITUNG ZUR VERWENDUNG IN PULVERINHALATOREN
COMPOSITION PHARMACEUTIQUE POUR L'UTILISATION AVEC UN INHALATEUR DE POUDRE SECHE

(30) Priority: 03.09.2010 EP 10175314
(43) Date of publication of application: 10.07.2013
(73) Proprietor: Pharmaterials Ltd., Reading, Berkshire RG2 0NH (GB)
(72) Inventor: NYAMBURA, Bildad Kimani, Reading RG2 0NH (GB)
(74) Representative: Jones, Helen M.M.
(86) International application number: PCT/EP2011/065330
(87) International publication number: WO 2012/028745

(56) References cited:
- WO-A1-99/29320
- WO-A1-03/103633
- WO-A1-2007/068443
- WO-A2-01/78695
- WO-A2-2006/124446
- WO-A2-2009/050217
- US-A1- 2004 105 822

## Description

The present invention relates to pharmaceutical formulations suitable for use in a dry powder inhaler which comprise carrier particles, active particles, and excipient wherein the excipient comprises phytosterol, phytosterol derivative, phytostanol, phytostanol derivative and combinations thereof. The invention also relates to a method of formulating said pharmaceutical formulations. The invention also relates to the use of said pharmaceutical formulations in a dry powder inhaler.

It is known to use excipient in combination with carrier particles and active particles in dry powder formulations. WO2005/004852 describes the use of calcium stearate to inhibit or reduce chemical interaction between an active ingredient substance and a carrier in a solid pharmaceutical formulation, wherein said active ingredient substance is susceptible to chemical interaction with said carrier.

US2010/0015238 discloses a pharmacological powder for inhalation comprising fine particles of a drug and particles of a force-controlling agent, wherein the particles of said force-controlling agent are disposed on the surface of the active particles as these are a particulate coating or a continuous or discontinuous film. It is therefore known to use force-controlling agents to coat active particles.

It is known to use magnesium stearate in dry powder inhalers. US7186401 discloses the use of pulverulent magnesium stearate in an amount of 0.1 to 2% by weight, based on the total weight of the formulation, said amount being effective to provide the Fine Particle Fraction (FPF) with reduced sensitivity to penetrating moisture and to stabilise the dry powder formulation. The FPF is the amount of the drug reaching the target area of the lung relative to the total released drug.

WO2004/093848 relates to enhancing the dosing efficiency of pharmaceutical dry powder formulations administered by pulmonary inhalation. It discloses known additive materials, or force-control agents (FCA) which have physical and chemical properties which lead to an enhanced FPF. The FCAs usually consist of physiologically acceptable material, although the FCAs may not always reach the lung. For example, where additive particles are attached to the surface of carrier particles, they will generally be deposited, along with those carrier particles at the back of the throat of the user. This Application says that FCA may be metal stearate, amino acid, phospholipids or cholesterol.

WO2009/050217 discloses compositions for use in a dry powder inhaler. Active particles are pretreated with an excipient, such as cholesterol and then the particles are mixed with a carrier.

WO2006/124446 discloses a composition of microparticles for delivery to the pulmonary system which provides sustained release of a pharmaceutical agent. The microparticles comprise a lipid structural matrix, which may include cholesterol or ergosterol. The liquid matrix at least partially encapsulates the pharmaceutical agent to allow controlled release of the pharmaceutical agent.

To the best of the inventor's knowledge, phytosterol, phytosterol derivative, phytostanol, phytostanol derivatives and combinations thereof have not been used in pharmaceutical compositions suitable for use in a dry powder inhaler.

It is desirable to reduce, or prevent any of the excipient from reaching the lung. Whilst studies into the effects of, for example magnesium stearate in the lung have not been identified by the inventors, it is believed that it is undesirable for this often used excipient to reach the lung.

There is a need therefore for a physiologically safe excipient to be used in dry powder inhaler compositions. There is a need for an excipient which does not have an adverse effect on the recipient.

Further there is a need for a method of making such compositions, to prevent the majority of the excipient from reaching the lung.

There is a need to improve the FPF of drug reaching the lung and to improve the storage stability of pharmaceutical compositions suitable for use in a dry powder inhaler.

### Summary of the Invention

In a first aspect of the invention, there is provided a pharmaceutical composition suitable for use in a dry powder inhaler comprising:
(i) carrier particles;
(ii) active particles adhered on the surface of the carrier particles for release from the carrier particles after inhalation in the respiratory tract; and
(iii) wherein the carrier particles have an excipient adhered on the surface for controlling the level of adhesion of the active particles, characterised in that the excipient comprises phytosterol, phytosterol derivative, phytostanol, phytostanol derivative or combinations thereof.

In a second aspect of the invention, there is provided a method of making a pharmaceutical composition suitable for use in a dry powder inhaler comprising:
(i) providing carrier particles;
(ii) providing active particles;
(iii) providing excipient, wherein the excipient comprises phytosterol, phytosterol derivative, phytostanol, phytostanol derivative or combinations thereof;
(iv) applying the excipient to the carrier particles;
(v) applying the active particles to the carrier particles;
(vi) recovering the resulting particles.

In a third aspect of the invention, there is provided a dry powder inhaler containing a composition according to the first aspect, or a composition produced according to the second aspect of the invention.

In a fourth aspect of the invention, there is provided said composition for use as a medicament treating a patient wherein the patient actuates the dry powder inhaler of the third aspect of the invention and inhales the pharmaceutical composition.

### Detailed Description

### Phytosterols and Phytostanol

It is preferred to reduce any adverse effects of the excipient in the lung. One way of doing this, as per the invention is to choose an excipient which is suitable for delivery to the lung.

Phytosterols and phytostanol are a large group of compounds that are found exclusively in plants. They are structurally related to cholesterol but differ from cholesterol in the structure of the side chain. They consist of a steroid skeleton with a hydroxyl group attached to the C-3 atom of the A-ring and an aliphatic side chain attached to the C-17 atom of the D-ring. Phytosterols have a double bond, typically between C-5 and C-6 of the sterol moiety, whereas this bond is saturated in phytostanols. This is shown in Figure I.

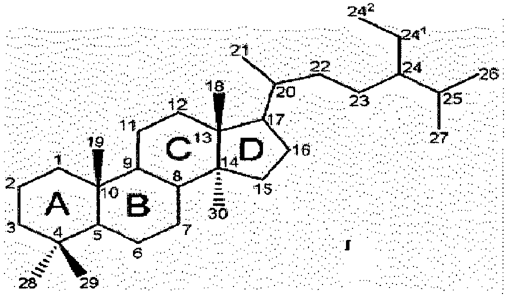

Below we show the structure of two phytosterols and two phytostanols.

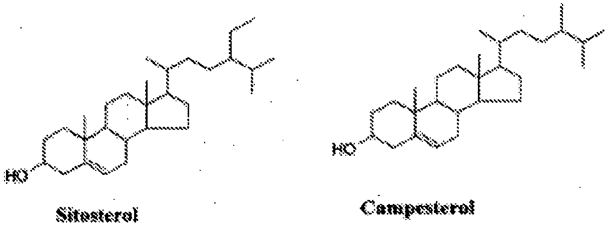

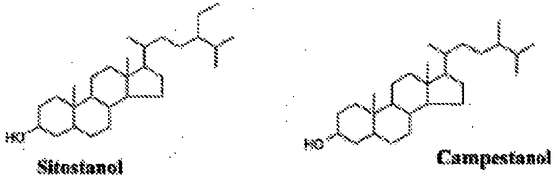

The most common phytosterols and phytostanols (examples of structures are shown above) are sitosterol (3ß-stigmast-5-en-3ol; CAS number 83-46-5), sitostanol (3ß,5α-stigmastan-3-ol; CAS number 83-45-4), campesterol (3ß,5α-Ergost-S-en-3-ol; CAS number 474-62-4), campestanol (3ß,5α-ergostan-3-ol; CAS number 474-60-2), stigmasterol (3ß-stigmasta-5,22-dien-3-ol; CAS number 83-48-7) and brassicasterol (3ß-ergosta-5,22-dien-3-ol; CAS number 474-67-9). Each commercial source has its typical phytosterols composition.

Commercially, phytosterols are isolated from vegetable oils, such as soybean oil, rapeseed (canola) oil, sunflower oil or com oil, or from so-called "tall oil", a by-product of the manufacture of wood pulp. Phytosterols can be hydrogenated to obtain phytostanols. Phytosterols and phytostanols are high melting powders:

In the present invention, the excipient preferably comprises sitosterol, sitostanol, campesterol, campestanol, stigmasterol, brassicasterol or combinations thereof, preferably sitosterol.

Derivatives of phytosterols and phytostanols include esters, preferably acetates such as stigmasterol acetate and sitosterol acetate.

Phytosterols and phytostanols and their derivatives are present in many food substances with the use of lowering cholesterol. These substances may include margarine, yoghurt drinks, olive oil, pasta and snack bars. They are generally regarded as safe (GRAS) by FDA.

The advantage of using these compositions is that they are used in food substances and therefore are used in the body and are generally regarded as safe. They are not believed to cause damage to the lungs if they are deposited there. They do not adversely affect the patient. Cholesterol and its derivatives are not used as the continued uptake of cholesterol can cause undesirable physiological effects to the patient. Further, as shown by Example 2, using a phytosterol or phytostanol such as sitosterol as an excipient results in a higher fine particle fraction of the active particles than using cholesterol as an excipient.

### Carrier Particles

The carrier particles preferably comprise lactose, maltose, sucrose, glucose or mixtures thereof, more preferably lactose, even more preferably lactose monohydrate.

Lactose is preferred because this has been used in many formulations in the past.

The geometric median size of the carrier particles measured by laser diffraction is preferably less than 150 µm, preferably less than 100 µm. Preferably the size range of the geometric median size is 30 to 150 µm, preferably 50 to 100 µm such as 60, 70, 84 or 95 µm.

The surface of the carrier particle is not usually smooth but has asperities and clefts in its surface. Without being bound by theory, it is believed that the site of an asperity or of a cleft is an area of high surface energy. The active particles are preferentially attracted to and adhere most strongly to these high energy sites causing uneven and reduced deposition of the active particles on the carrier surface. If an active particle adheres to a high energy site, it is subjected to a greater adhesion force than a particle at a lower energy site on the carrier particle and will therefore be less likely to be able to leave the surface of the carrier particle on actuation of the inhaler and be dispersed in the respiratory tract. It is therefore highly advantageous to reduce the number of these high energy sites by using the excipient.

The weight ratio of the excipient to the carrier particles is in the range 0.5:99.5 to 50:50, preferably 5:95 to 40:60, most preferably 10:90 to 30:70.

### Active Particles

The active particles of the present invention may be chosen from beta-mimetics such as Levalbuterol, Terbutalin, Reproterol, Salbutamol, Salmeterol, Formoterol, Fenoterol, Clenbuterol, Bambuterol, Tulobuterol, Broxaterol, Indacaterol, Epinephrin, Isoprenaline or Hexoprenaline; an Anticholinergic such as Tiotropium, Ipratropium, Oxitropium or Glycopyrronium; a Corticosteroid; such as Butixocort, Rofleponide, Budesonide, Ciclesonide, Mometasone, Fluticasone, Beclomethasone, Loteprednol or Triamcinolone; a Leukotriene antagonist, such as Andolast, Iralukast, Pranlukast, Imitrodast, Seratrodast, Zileuton, Zafirlukast or Montelukast; a Phosphodiesterase-Inhibitor, such as Filaminast or Piclamilast; an PAF-Inhibitor, such as Apafant, Forapafant or Israpafant; a potassium channel opener such as Amiloride or Furosemide; a pain killer such as Morphine, Fentanyl, Pentazocine, Buprenorphine, Pethidine, Tilidine, Methadone or Heroin; a potency agent such as Sildenafil, Alprostadil or Phentolamine; or a pharmaceutically acceptable derivative or salt of any of the foregoing compounds or classes of compounds. In as much as any of these compounds possess chiral centres, the compounds can be used in optically pure form, or can be presented as diastereomeric mixtures or racemic mixtures. Dry powders of the present invention may also employ proteins, peptides, oligopeptides, polypeptides, polyamino acids, nucleic acids, polynucleotides, oligo-nucleotides and high molecular weight polysaccharides. Examples of macromolecules that find use in the present invention are: Albumins (preferably, human serum Insulin; albumin); BSA; IgG; IgM; insulin; GCSF; GMCSF; LHRH; VEGF; hGH; lysozyme; alpha-lactoglobulin; basic fibroblast growth factor; (bFGF); asparaginase; tPA; urokinase-VEGF; chymotrypsin; trypsin; streptokinase; interferon; carbonic anhydrase; ovalbumin; glucagon; ACTH; oxytocin; phosphorylase b; alkaline phosphatase-secretin; vasopressin; levothyroxin; phatase; beta-galactosidase; parathyroid hormone, calcitonin; fibrinogen; polyaminoacids (e.g., DNAse, alpha 1 antitrypsin; polylysine, polyarginine); angiogenesis inhibitors or pro-immunoglobulins (e.g., antibodies); motors; somatostatin and analogs; casein; collagen; gelatin; soy protein; and cytokines (e.g., interferon, interleukin); immunoglobulins.

Physiologically active proteins such as peptide hormones, cytokines, growth factors, factors acting on the cardiovascular system, factors acting on the central and peripheral nervous systems, factors acting on humoral electrolytes and hemal substances, factors acting on bone and skeleton, factors acting on the gastrointestinal system, factors acting on the immune system, factors acting on the respiratory system, factors acting on the genital organs, and enzymes.

Hormones and hormone modulators including insulin, proinsulin, C-peptide of insulin, a mixture of insulin and C-peptide of insulin, hybrid insulin cocrystals (Nature Biotechnology, 20, 800-804, 2002), growth hormone, parathyroid hormone, luteinizing hormone-releasing hormone (LH-RH), adrenocorticotropic hormone (ACTH), amylin, oxytocin, luteinizing hormone, (D-Tryp6)-LHRH, nafarelin acetate, leuprolide acetate, follicle stimulating hormone, glucagon, prostaglandins, estradiols, testosterone, and other factors acting on the genital organs and their derivatives, analogues and congeners. As analogues of said LH-RH, such known substances as those described in U.S. Pat. Nos. 4,008,209, 4,086,219, 4,124,577, 4,317,815 and 5,110,904 can be mentioned.

Hematopoietic or thrombopoietic factors include, among others, erythropoietin, granulocyte colony stimulating factor (G-CSF), granulocyte-macrophage stimulating factor (GM-CSF) and macrophage colony stimulating factor (M-CSF), leukocyte proliferation factor preparation (Leucoprol, Morinaga Milk), thrombopoietin, platelet proliferation stimulating factor, megakaryocyte proliferation (stimulating) factor, and factor VIII.

Therapeutic factors acting on bone and skeleton and agents for treating osteoporosis including bone GLa peptide, parathyroid hormone and its active fragments (osteostatin, Endocrinology 129, 324, 1991), histone H4-related bone formation and proliferation peptide (OGP, The EMBO Journal 11, 1867, 1992) and their muteins, derivatives and analogs thereof.

Enzymes and enzyme cofactors including pancrease, L-asparaginase, hyaluronidase, chymotrypsin, trypsin, tPA, streptokinase, urokinase, pancreatin, collagenase, trypsinogen, chymotrypsinogen, plasminogen, streptokinase, adenyl cyclase, and superoxide dismutase (SOD).

Vaccines include Hepatitis B, MMR (measles, mumps, and rubella), and Polio vaccines.

Growth factors include nerve growth factors (NGF, NGF-2/NT-3), epidermal growth factor (EGF), fibroblast growth factor (FGF), insulin-like growth factor (IGF), transforming growth factor (TGF), platelet-derived cell growth factor (PDGF), and hepatocyte growth factor (HGF).

Factors acting on the cardiovascular system including factors which control blood pressure, arteriosclerosis, etc., such as endothelins, endothelin inhibitors, endothelin antagonists described in EP 436189, 457195, 496452 and 528312, JP [Laid Open] No. H-3-94692/1991 and 130299/1991, endothelin producing enzyme inhibitors, vasopressin, renin, angiotensin I, angiotensin II, angiotensin III, angiotensin I inhibitor, angiotensin II receptor antagonist, atrial natriuretic peptide (ANP), and antiarrhythmic peptide; Factors acting on the central and peripheral nervous systems including opioid peptides (e.g. enkephalins, endorphins), neurotropic factor (NTF), calcitonin gene-related peptide (CGRP), thyroid hormone releasing hormone (TRH), salts and derivatives of TRH [JP [Laid Open] No. 50-121273/1975 (U.S. Pat. No. 3,959,247), JP [Laid Open] No. 52-116465/1977 (U.S. Pat. No. 4,100,152)], and neurotensin.

Factors acting on the gastrointestinal system including secretin and gastrin.

Factors acting on humoral electrolytes and hemal substances including factors which control hemagglutination, plasma cholesterol level or metal ion concentrations, such as calcitonin, apoprotein E and hirudin. Laminin and intercellular adhesion molecule 1 (ICAM 1) represent exemplary cell adhesion factors.

Factors acting on the kidney and urinary tract including substances which regulate the function of the kidney, such as brain-derived natriuretic peptide (BNP), and urotensin.

Factors which act on the sense organs including factors which control the sensitivity of the various organs, such as substance P.

Chemotherapeutic agents, such as paclitaxel, mytomycin C, BCNU, and doxorubicin.

Factors acting on the immune system including factors which control inflammation and malignant neoplasms and factors which attack infective microorganisms, such as chemotactic peptides and bradykinins; and

Naturally occurring, chemically synthesized or recombinant peptides or proteins which may act as antigens, such as cedar pollen and ragweed pollen, and these materials alone or together with coupled to haptens, or together with an adjuvant.

The present invention is particularly useful in the formulation of hydrophilic and moisture sensitive active substances, such as the salt forms of any of the compounds mentioned above such as the chloride, bromide, iodide, nitrate, carbonate, sulphate, methylsulphate, phosphate, acetate, benzoate, benzenesulfonate, fumarate, malonate, tartrate, succinate, citrate, lactate, gluconate, glutamate, edentate, mesylate, pamoate, pantothenate or hydroxynaphthoate; or an ester form such as an acetate, propionate, phosphate, succinate or etabonate.

Preferably the active particles comprise corticosteroids such as beclomethasone, budesonide and fluticasone, ß₂-agonists such as salbutamol, formoterol, salmeterol and fenoterol; or anticholingergic agents such as atropine, benzatropine, glycopyrrolate and ipratropium bromide; peptides or proteins, such as an antibody, enzyme or a hormone; nucleic acids, such as siRNA; or DNA such as gene therapy vectors and gene vaccines and pharmaceutically acceptable salts, esters, hydrates or solvates thereof. In particular, the active particles can comprise fluticasone propionate, salmeterol xinafoate, or salbutamol sulphate.

Preferably the MMAD of the active particles is in the range 1 µm to 10 µm, preferably 2 µm to 7 µm, most preferably 3 µm to 5 µm. These sizes are required so that the active particles reach the lungs. If the active particles are too large, they will be deposited before they reach the lung, for example in the throat or in the device. If the active particles are too small, they could simply be exhaled.

### Composition

The pharmaceutical composition suitable for use in a dry powder inhaler comprises
(i) carrier particles;
(ii) active particles on the surface of the carrier particles; and
(iii) excipient on the surface of the carrier particles, wherein the excipient comprises phytosterol, phytosterol derivative, phytostanol, phytostanol derivative or combinations thereof.

The composition comprises the preferred features of the carrier particles, active particles and excipient described above.

It is known for lactose to react with a drug particle, sometimes in what is known as a Malliard reaction. This reaction is undesirable and it is an advantage of the present invention that the use of the composition of the present invention reduces the speed and extent of the Malliard reaction when compared to using no excipient. This will increase the storage time of the composition of the invention compared to using no excipient.

The storage life of a pharmaceutical composition used in a dry particle inhaler is also dependent on the fine particle fraction (FPF). The FPF is the actual amount of the drug that reaches the target area in the lung. The FPF of embodiments of the composition of the present invention over time do not vary as much as the FPF of a composition which does not comprise an excipient. This is important for the storage life of a dry powder composition.

### Method

A method of making a pharmaceutical composition suitable for use in a dry powder inhaler comprising:
(i) providing carrier particles;
(ii) providing active particles;
(iii) providing excipient, wherein the excipient comprises phytosterol, phytosterol derivative, phytostanol, phytostanol derivative or combinations thereof;
(iv) applying the excipient to the carrier particles;
(v) applying the active particles to the carrier particles;
(vi) recovering the resulting particles.

In the above method, steps (i) to (iii) may be performed in any order. Steps (iv) and (v) may be performed in any order. Additionally, the particles may be treated with heat, such as in an oven, and/or be subjected to low pressure in order to evaporate the solvent.

In a preferred embodiment, step (iv) comprises:
(a) providing a solvent, wherein the excipient is soluble in the solvent and the carrier particles are insoluble in the solvent,
(b) dissolving the excipient in the solvent to form a solution;
(c) adding the carrier particles to the solution;
(d) optionally applying ultrasound; and
(e) drying the carrier particles to form carrier particles with excipient on the surface.

"Soluble" means that the substance, in this case the excipient, is soluble in an amount of greater than 0.1 mg per ml at 25°C, preferably greater than 0.5 mg per ml at 25°C, more preferably greater than 5 mg per ml at 5°C.

"Insoluble" means that the substance, in this case the carrier particles, is soluble in amount of less than 0.1 mg per ml at 25°C, preferably less than 0.01 mg per ml at 25°C, more preferably less than 0.001 mg per ml at 25°C.

In step b, dissolving the excipient means that at least 50 wt% of the excipient is dissolved into the solvent, preferably at least 80 wt% of the excipient is dissolved in the solvent, most preferably at least 95 wt% of the excipient dissolved in the solvent, ideally substantially all of the excipient is dissolved in the solvent. The solvent may be heated to allow more of the excipient to dissolve in it, such as up to at least 40°C, or at least 50°C, or at least 80°C.

The solvent is preferably dichloromethane, acetone or ethanol, most preferably dichloromethane as these solvents have low boiling points are readily evaporate.

In step d, ultrasound is optionally applied in order to ensure that the carrier particles are not agglomerated. Ultrasound can be applied to ensure thorough dispersion and coating of the carrier particles in the suspension.

In step e, the carrier particles may be dried by filtering, following by treatment in an oven. Alternatively, the particles may be dried by using vacuum, spray or freeze drying.

In step v, applying the active particles to the carrier particles preferably involves applying the active particles in particulate form. Preferably the active particles are not dissolved in the solvent prior to application to the carrier particles. When preferred steps a to e are carried out, step iv is carried out before step v, that is the excipient is applied to the carrier particles, prior to the active particles being applied to the carrier particles.

Step iv and step v could be carried out by air-jet milling, stirring, mechanofusion, low or high shear mixing, co-grinding or mixtures thereof.

It is preferred to reduce the amount of excipient that is delivered to the lung. The inventors have also found that according to the method of the invention, by first dissolving the excipient, and then adding the carrier particles to form carrier particles with excipient on the surface of the carrier particles, the excipient is much better adhered to the carrier particle than by simple mixing or micronisation as known in the art.

Whilst not wishing to be limited by theory, it is proposed that the step of forming the excipient on the surface of the carrier particles by the method described above means that more of the excipient is in the high energy sites on the carrier. The preferred embodiment of adding the excipient from solution onto the carrier particle provides for improved adhesion to the carrier and has the advantage of reducing the amount of excipient that is delivered to the lung. Preferably, less than 20% by weight of the excipient reaches the lung, more preferably less than 10%, more preferably less than 5% by weight, more preferably less than 1% by weight reached the lung, most preferably no excipient reached the lung.

### Dry Powder Inhaler

The composition as described above, and the compositions made by the method described above may be loaded in a dry powder inhaler. The inhaler may be a single dose or a multi dose inhaler.

A further embodiment of the invention is a method of treating a patient wherein the patient actuates the dry powder inhaler containing the composition of the invention and inhales the pharmaceutical composition. The method of treatment could be for treating local and systemic indications such as asthma, COPD, bronchitis, cystic fibrosis, lung cancer, diabetes and migraines.

The invention now will be described by the following example which does not limit the scope of the invention.

### Example 1

### Lactose Coating

50 ml of 10% w/v phytosterols in acetone solution was prepared. The phytosterol used was CardioAid™. CardioAid contains a minimum of 95% plant sterols. The composition comprises 40-58% beta-sitosterol, 20-30% campesterol, 14-22% stigmasterol, 0-6% brassicasterol and 0-5% sitostanol. 10g of lactose monohydrate (DMV Fonterra grade: SV003 grade) was suspended in the solution containing phytosterols and the suspension was sonicated for 5 minutes. Lactose monohydrate was subsequently filtered and dried in an oven set at 40°C for 12 hours to generate lactose monohydrate material coated with thin layer of phytosterols.

### Powder Blend Preparation

### Comparative composition

Powder blend containing lactose monohydrate and salbutamol sulphate. 100 mg of micronized salbutamol sulphate was weighed into a glass vial. 4900 mg of lactose monohydrate (DMV Fonterra grade SV003 grade) was added and the content was thoroughly mixed using a spatula for 5 minutes. The content was subsequently mixed using Turbula^{®} mixer for 10 minutes to produce homogeneous powder blend lactose monohydrate and micronized salbutamol sulphate.

### Composition 1

The procedure for the comparative composition described above, was repeated with the lactose monohydrate fraction being substituted with coated lactose monohydrate prepared as described above.

### Composition 2

4410mg of lactose monohydrate (SV003, DMV Fonterra grade) was weighed into a glass vial. 490mg of coated lactose monohydrate was added into the vial and the content mixed thoroughly using a spatula for 5 minutes. The content was subsequently mixed using a Turbula^{®} mixer for 10 minutes to produce an homogeneous powder blend of lactose monohydrate and coated lactose monohydrate.

100mg of micronized salbutamol sulphate was weighed and subsequently added into the vial containing the lactose monohydrate and coated lactose monohydrate powder blend. The content was mixed thoroughly using a spatula for 5 minutes followed by mixing using a Turbula^{®} mixer for 10 minutes to produce an homogeneous powder blend of lactose monohydrate, coated lactose monohydrate and micronized salbutamol sulphate (i.e. composition 2).

Composition 2 was tested for aerodynamic particle size distribution using a NGI (day 1 data). The composition was subsequently stored at 40°C/75%RH for a period of 1 month and the aerodynamic particle size distribution was determined again using the NGI.

### Determination of Aerodynamic Particle Size Distribution Using NGI Apparatus

### Equipment

- NGI (Copley Scientific, UK) with impaction cups
- USP throat and pre-separator
- Rubber mouthpiece for positioning/sealing DPI device
- Pump capable of drawing 60L/minute

### Preparation of Coating Solution

100 mg of Pluronic F68 (surfactant) was weighed and put into a clean 100 ml volumetric flask. 3 ml of PEG 600 was added and the content was made up to volume using acetone. The content was thoroughly mixed.

### Assembly of NGI Apparatus

The NGI was washed with methanol, rinsed with acetone and allowed to dry prior to analysis. The coating solution was added into the cups as illustrated in Table 1 and acetone was allowed to evaporate to dryness. The apparatus was assembled and connected to the flow regulator and vacuum pump. 10 ml of distilled water was added in the pre-separator. The airflow through the apparatus, as measured at the inlet to the throat, was adjusted to the desired air flow rate of 60 L/min. A molded rubber adapter was placed in position at the end of the throat piece such that the DPI device, when fitted into the mouthpiece, will be aligned along the vertical axis of the throat piece.

**Table 1: Volume of coating solution added to NGI cups**

| **Stage** | **Coating solution (ml)** |
|---|---|
| 1 | 3 |
| 2-7 | 1.5 |
| Micro-orifice collector (MOC) | 3 |

### Dose Dispersion Through the NGI Apparatus

The cyclohaler device was opened and a single capsule containing the composition was put into the capsule chamber. The device was closed and the capsule pierced. The device was fitted to the mouthpiece adapter and vacuum flow activated. After 4 seconds, the airflow through the device and apparatus was stopped. This procedure was repeated until 10 doses were discharged through the NGI apparatus

### Preparation and Analysis of Sample Solutions

The sample preparation volumes are described in Table 2. Each stage was washed with dissolving solvent (70% v/v methanol in water) into the appropriate size of volumetric flask. The samples were analysed using HPLC. This procedure was carried out for composition 1, composition 2 and the comparative composition within 24 hours of making the blend. The results are shown in Table 3.

Part of the comparative composition and composition 2 was stored for 1 month at accelerated stability conditions (40°C and 75%RH). The results are shown in Table 4.

**Table 2: Sample volumes**

| **Stage** | **Flask Size (ml)** |
|---|---|
| Throat (including mouthpiece) | 50 |
| Pre-separator | 100 |
| 1-external filter | 50 |

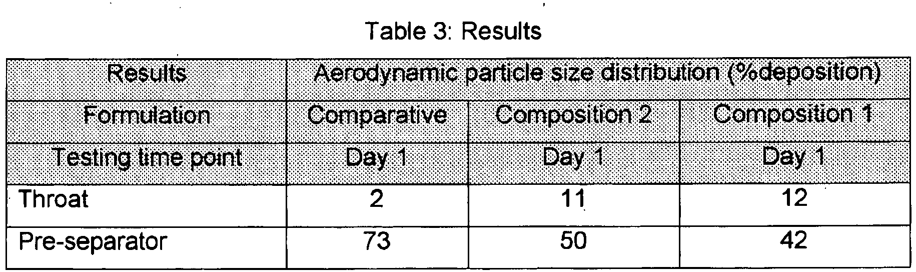

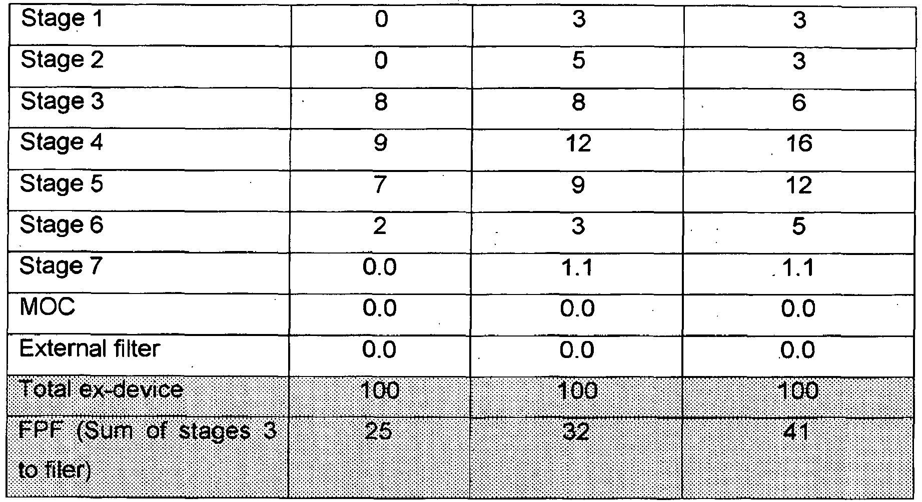

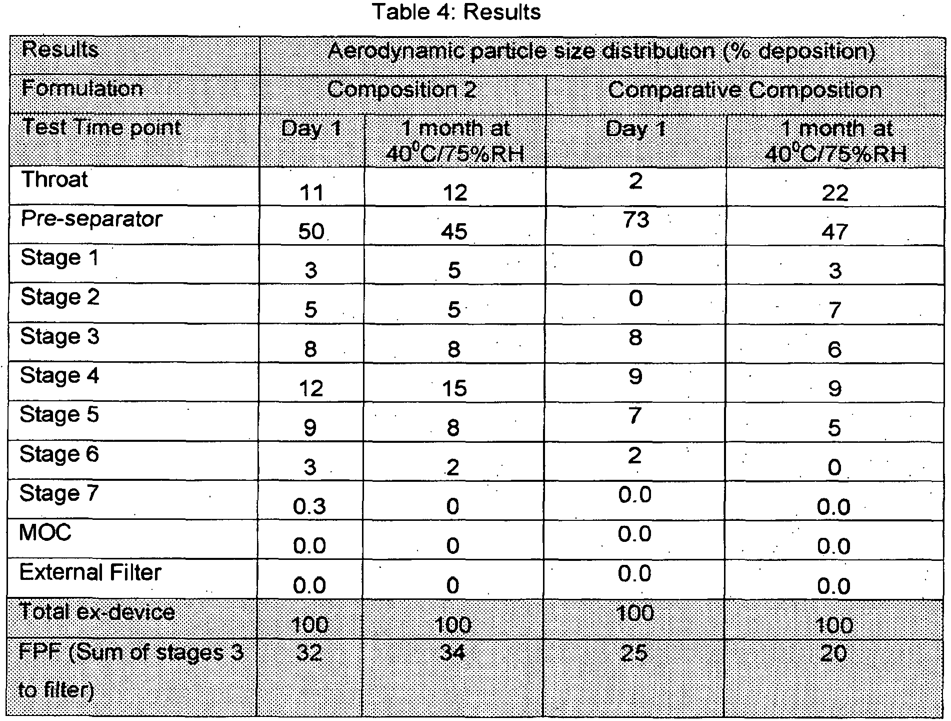

As shown by the results above, there is improved FPF deposition when the lactose has been coated with phytosterols. Composition 1 has the highest FPF and the comparative Composition has the lowest FPF

Composition 2, whilst not delivering the same FPF as composition 1, has an enhanced FPF compared to the comparative composition and has improved physical stability. The data show an improved formulation which is protected from humidity induced physical instability. This is shown by the different FPF between storage after one day and storage after one month under accelerated stability conditions of 40°C 75% RH of comparative composition and composition 2. The data show that even when a proportion of the lactose has been coated with phytosterols, there is improved FPF. It is desirable that the physical stability of the formulation is improved for storage purposes.

This shows the benefit of the phytosterols in both improving the FPF deposition and physical stability by appropriate use of the invention.

It is known for lactose to react with a drug particle, sometimes in what is known as a Malliard reaction and it is believed that this formulation improves the chemical stability of the formulation.

### Example 2

### Preparation of coating solution

250mg of each of sitosterol and cholesterol were weighed into two different beakers. 50ml of Dichloromethane (DCM) was added to each of the two beakers and the solids dissolved to make 5% solution of each of sitosterol and cholesterol. These solutions were used to coat lactose.

### Lactose coating

20g of lactose was weighed and added into each beaker containing the coating solution of sitosterol or cholesterol. A control sample was prepared by adding 20g of lactose into a third beaker containing 50ml DCM only.

The contents in each beaker were stirred using a stirring rod for 5 minutes and then covered and then the content was further sonicated for 10 minutes using a Grant Ultrasonic water bath. The content was allowed to stand at room temperature for 30 minutes to allow lactose to settle at the bottom. The supernatant liquid was subsequently decanted and the lactose sample recovered separately from each beaker. These samples were kept in the oven set at 40°C overnight. The dried samples were collected separately into labelled amber glass bottles and kept in ambient conditions in the cupboard. This resulted with:
1. Control lactose carrier
2. Lactose coated with sitosterol carrier
3. Lactose coated with cholesterol carrier

### Preparation of powder blends

Powder blends containing compositions below were prepared.

**Table 5: Powder blend composition**

| Powder blend | Composition (mg) | | | | | |
|---|---|---|---|---|---|---|
| | Carrier | | | Active Pharmaceutical Ingredient (API) | | |
| | Control lactose | Lactose coated with sitosterol | Lactose coated with cholesterol | Micronized salmeterol xinafoate (SX) | Micronised fluticasone propionate (FP) | Total |
| 1 | 4987.5 | 0 | 0 | 12.5 | 0 | 5000 |
| 2 | 4987.5 | 0 | 0 | 0 | 12.5 | 5000 |
| 3 | 0 | 4987.5 | 0 | 12.5 | 0 | 5000 |
| 4 | 0 | 4987.5 | 0 | 0 | 12.5 | 5000 |
| 5 | 0 | 0 | 4987.5 | 12.5 | 0 | 5000 |
| 6 | 0 | 0 | 4987.5 | 0 | 12.5 | 5000 |

Micronized API was weighed into a glass vial in each experiment. Carrier lactose in each experiment was added and the content was thoroughly mixed using a spatula for 5 minutes. The content was subsequently mixed using Turbula® mixer for 10 minutes to produce homogeneous powder blend of carrier lactose and micronized API.

The powder blends were stored at 40°C/75%RH conditions for a period of two weeks and the aerodynamic particle size distribution was determined using the method described in Example 1.

### Results

The aerodynamic particle size distribution results are shown in Table 6 & 7.

**Table 6**

| **Results** | **% deposition** | | |
|---|---|---|---|
| **API** | **Fluticasone propionate (FP)** | | |
| **Stage** | **Lactose** | **Lactose -Sitosterol** | **Lactose -Cholesterol** |
| Throat | 28.121 | 24.412 | 26.873 |
| Pre-separator | 59.556 | 60.101 | 59.473 |
| Stage1 | 2.453 | 2.047 | 2.291 |
| Stage2 | 1.625 | 1.894 | 1.982 |
| Stage3 | 2.387 | 3.608 | 3.301 |
| Stage4 | 3.439 | 4.668 | 3.750 |
| Stage5 | 1.239 | 2.188 | 1.476 |
| Stage6 | 0.532 | 0.549 | 0.457 |
| Stage7 | 0.648 | 0.533 | 0.397 |
| MOC | 0.000 | 0.000 | 0.000 |
| **FPF (3 - 7)** | **8.245** | **11.545** | **9.381** |

**Table 7**

| **Results** | **% deposition** | | |
|---|---|---|---|
| **API** | **Salmeterol xinafoate (SX)** | | |
| **Stage** | **Lactose** | **Lactose - Sitosterol** | **Lactose** - **Cholesterol** |
| Throat | 17.719 | 13.068 | 12.261 |
| Pre-separator | 77.394 | 76.654 | 77.270 |
| Stage 1 | 1.442 | 1.573 | 2.513 |
| Stage 2 | 0.468 | 0.769 | 1.362 |
| Stage 3 | 0.665 | 1.308 | 1.525 |
| Stage 4 | 1.327 | 3.507 | 2.811 |
| Stage 5 | 0.857 | 2.464 | 1.552 |
| Stage 6 | 0.127 | 0.657 | 0.565 |
| Stage 7 | 0.000 | 0.000 | 0.142 |
| MOC | 0.000 | 0.000 | 0.000 |
| **FPF(3-7)** | **2.976** | **7.936** | **6.594** |

The results for both FP and SX show that %FPF deposition was improved when lactose coated with cholesterol and sitosterol was used in the formulation compared with the control lactose. Lactose coated with sitosterol demonstrated the highest FPF deposition, thus demonstrating the advantage of using sitosterol instead of cholesterol to coat the lactose.

## Claims

1. A pharmaceutical composition suitable for use in a dry powder inhaler comprising a powder formed of:
(i) carrier particles;
(ii) active particles adhered on the surface of the carrier particles for release from the carrier particles after inhalation in the respiratory tract; and
(iii) wherein the carrier particles have an excipient adhered on the surface for controlling the level of adhesion of the active particles, **characterised in that** the excipient comprises phytosterol, phytosterol derivative, phytostanol, phytostanol derivative or combinations thereof.

2. The composition according to any preceding claim wherein the excipient comprises sitosterol, sitostanol, campesterol, campestanol, stigmasterol, brassicasterol or combinations thereof.

3. The composition according to any preceding claim wherein the weight ratio of the excipient to the carrier particles is in the range 0.5:99.5 to 50:50, preferably 5:95 to 40:60.

4. The composition according to any preceding claim wherein the carrier particles comprise lactose, maltose, sucrose, glucose or mixtures thereof, preferably lactose.

5. The composition according to any preceding claim wherein the geometric median size of the carrier particles is less than 150µm, preferably less than 100µm.

6. The composition according to any preceding claim wherein the active particles comprise corticosteroids such as beclomethasone, budesonide and fluticasone, ß₂-agonists such as salbutamol, formoterol, salmeterol and fenoterol or anticholinergic agents such as atropine, benzatropine, glycopyrrolate and ipratropium bromide.

7. The composition according to any preceding claim wherein the MMAD of the active particles is in the range 1µm to 10µm, preferably 2µm to 7µm, most preferably 3µm to 5µm.

8. A method of making a pharmaceutical composition suitable for use in a dry powder inhaler comprising:
(i) providing carrier particles;
(ii) providing active particles;
(iii) providing excipient, wherein the excipient comprises phytosterol, phytosterol derivative, phytostanol, phytostanol derivative or combinations thereof;
(iv) applying the excipient to the carrier particles;
(v) applying the active particles to the carrier particles;
(vi) recovering the resulting particles.

9. The method according to claim 8 wherein step (iv) comprises:
1. providing a solvent, wherein the excipient is soluble in the solvent and the carrier particles are insoluble in the solvent,
2. dissolving the excipient in the solvent to form a solution;
3. adding the carrier particles to the solution;
4. optionally applying ultrasound; and
5. drying the carrier particles to form carrier particles with excipient on the surface.

10. The method according to claim 8 wherein applying comprises air-jet milling, stirring, mechanofusion, low or high shear mixing, co-grinding or mixtures thereof.

11. A dry powder inhaler containing a composition according to any claims 1 to 7.

12. A pharmaceutical composition according to any of claims 1 to 7 for use as a medicament.

13. A pharmaceutical composition for use according to claim 12 in the treatment of asthma, COPD, bronchitis, cystic fibrosis, lung cancer, diabetes or migraines.

## Patentansprüche

1. Eine pharmazeutische Zusammensetzung, die zur Verwendung in einem Trockenpulverinhalator geeignet ist, die ein Pulver beinhaltet, das gebildet ist aus:
(i) Trägerpartikeln;
(ii) wirksamen Partikeln, die auf der Oberfläche der Trägerpartikel haften, zur Freisetzung aus den Trägerpartikeln nach Inhalation im Atemtrakt; und
(iii) wobei die Trägerpartikel einen Hilfsstoff, der auf der Oberfläche haftet, zum Steuern des Grads der Haftung der wirksamen Partikel aufweisen, **dadurch gekennzeichnet, dass** der Hilfsstoff Phytosterol, Phytosterolderivat, Phytostanol, Phytostanolderivativ oder Kombinationen davon beinhaltet.

2. Die Zusammensetzung gemäß einem vorhergehenden Anspruch, wobei der Hilfsstoff Sitosterol, Sitostanol, Campesterol, Campestanol, Stigmasterol, Brassicasterol oder Kombinationen davon beinhaltet.

3. Die Zusammensetzung gemäß einem vorhergehenden Anspruch, wobei das Gewichtsverhältnis des Hilfsstoffs zu den Trägerpartikeln im Bereich von 0,5:99,5 bis 50:50 liegt, bevorzugt von 5:95 bis 40:60.

4. Die Zusammensetzung gemäß einem vorhergehenden Anspruch, wobei die Trägerpartikel Lactose, Maltose, Sucrose, Glucose oder Mischungen davon beinhalten, bevorzugt Lactose.

5. Die Zusammensetzung gemäß einem vorhergehenden Anspruch, wobei der geometrische Mediandurchmesser der Trägerpartikeln weniger als 150 µm, bevorzugt weniger als 100 µm, beträgt.

6. Die Zusammensetzung gemäß einem vorhergehenden Anspruch, wobei die wirksamen Partikel Folgendes beinhalten: Kortikosteroide wie etwa Beclomethason, Budesonid und Fluticason; β₂-Agonisten wie etwa Salbutamol, Formoterol, Salmeterol und Fenoterol; Anticholinergika wie etwa Atropin, Benzatropin, Glycopyrrolat und Ipratropiumbromid.

7. Die Zusammensetzung gemäß einem vorhergehenden Anspruch, wobei der MMAD der wirksamen Partikel im Bereich von 1 µm bis 10 µm, bevorzugt von 2 µm bis 7 µm, besonders bevorzugt von 3 µm bis 5 µm liegt.

8. Ein Verfahren zur Herstellung einer pharmazeutischen Zusammensetzung, die zur Verwendung in einem Trockenpulverinhalator geeignet ist, das Folgendes beinhaltet:
(i) Bereitstellen von Trägerpartikeln;
(ii) Bereitstellen von wirksamen Partikeln;
(iii) Bereitstellen von Hilfsstoff, wobei der Hilfsstoff Phytosterol, Phytosterolderivat, Phytostanol, Phytostanolderivat oder Kombinationen davon beinhaltet;
(iv) Applizieren des Hilfsstoffs auf die Trägerpartikel;
(v) Applizieren der wirksamen Partikel auf die Trägerpartikel;
(vi) Rückgewinnen der resultierenden Partikel.

9. Das Verfahren gemäß Anspruch 8, wobei Schritt (iv) Folgendes beinhaltet:
1. Bereitstellen eines Lösungsmittels, wobei der Hilfsstoff in dem Lösungsmittel löslich ist und die Trägerpartikel nicht in dem Lösungsmittel löslich sind,
2. Auflösen des Hilfsstoffs in dem Lösungsmittel, um eine Lösung zu bilden;
3. Zugeben der Trägerpartikel zu der Lösung;
4. optional Anwenden von Ultraschall; und
5. Trocknen der Trägerpartikel, um Trägerpartikel mit Hilfsstoff auf der Oberfläche zu bilden.

10. Das Verfahren gemäß Anspruch 8, wobei das Applizieren Luftstrahlmahlen, Rühren, Mechanofusion, Niedrig- oder Hochschermischen, Komahlen oder Mischungen davon beinhaltet.

11. Ein Trockenpulverinhalator, der eine Zusammensetzung gemäß einem der Ansprüche 1 bis 7 enthält.

12. Eine pharmazeutische Zusammensetzung gemäß einem der Ansprüche 1 bis 7 zur Verwendung als Medikament.

13. Eine pharmazeutische Zusammensetzung zur Verwendung gemäß Anspruch 12 bei der Behandlung von Asthma, COPD, Bronchitis, zystischer Fibrose, Lungenkrebs, Diabetes oder Migränen.

## Revendications

1. Composition pharmaceutique appropriée pour une utilisation avec un inhalateur de poudre sèche comprenant une poudre formée de :
(i) particules vectrices ;
(ii) particules actives qui adhèrent à la surface des particules vectrices et sont libérées des particules vectrices après inhalation dans les voies respiratoires ; et
(iii) dans laquelle un excipient adhérant à la surface des particules vectrices contrôle le degré d'adhésion des particules actives, **caractérisée en ce que** l'excipient comprend un phytostérol, le dérivé d'un phytostérol, un phytostanol, le dérivé d'un phytostanol ou des combinaisons de ceux-ci.

2. Composition selon l'une quelconque des revendications précédentes, dans laquelle l'excipient comprend le sitostérol, le sitostanol, le campestérol, le campestanol, le stigmastérol, le brassicastérol ou des combinaisons de ceux-ci.

3. Composition selon l'une quelconque des revendications précédentes, dans laquelle le rapport de poids entre l'excipient et les particules vectrices est dans la plage de 0,5:99,5 à 50:50, préférablement de 5:95 à 40:60.

4. Composition selon l'une quelconque des revendications précédentes, dans laquelle les particules vectrices comprennent le lactose, le maltose, le sucrose, le glucose ou des mélanges de ceux-ci, préférablement le lactose.

5. Composition selon l'une quelconque des revendications précédentes dans laquelle la taille géométrique médiane des particules vectrices est inférieure à 150 µm, préférablement inférieure à 100 µm.

6. Composition selon l'une quelconque des revendications précédentes, dans laquelle les particules actives comprennent des corticostéroïdes comme le béclométhasone, le budésonide et le fluticasone, des agonistes β₂-adrénergiques comme le salbutamol, le formotérol, le salmétérol et le fénotérol ou des agents anticholinergiques comme l'atropine, la benzatropine, le glycopyrrolate et le bromure d'ipratropium.

7. Composition selon l'une quelconque des revendications précédentes, dans laquelle le diamètre aérodynamique médian en masse (DAMM) des particules vectrices est dans la plage de 1 µm à 10 µm, préférablement de 2 µm à 7 µm et plus préférablement de 3 µm à 5 µm.

8. Procédé de fabrication d'une composition pharmaceutique appropriée pour une utilisation avec un inhalateur de poudre sèche comprenant :
(i) la préparation de particules vectrices ;
(ii) la préparation de particules actives ;
(iii) la préparation d'un excipient, l'excipient comprenant un phytostérol, le dérivé d'un phytostérol, un phytostanol, le dérivé d'un phytostanol ou des combinaisons de ceux-ci ;
(iv) l'application de l'excipient sur les particules vectrices ;
(v) l'application des particules actives sur les particules vectrices ;
(vi) la récupération des particules ainsi produites.

9. Procédé selon la revendication 8, dans lequel l'étape (iv) comprend :
1. l'utilisation d'un solvant, l'excipient étant soluble dans le solvant et les particules vectrices étant insolubles dans le solvant ;
2. la dissolution de l'excipient dans le solvant pour obtenir une solution ;
3. l'addition des particules vectrices à la solution ;
4. en option l'application d'ultrasons ; et
5. le séchage des particules vectrices pour former des particules vectrices portant l'excipient à la surface.

10. Procédé selon la revendication 8, dans lequel l'application comprend un broyage à jet d'air, une agitation, une mécanofusion, un mélange sous cisaillement faible ou élevé, un co-broyage ou des mélanges de ces techniques.

11. Inhalateur de poudre sèche contenant une composition selon l'une quelconque des revendications 1 à 7.

12. Composition pharmaceutique selon l'une quelconque des revendications 1 à 7 pour une utilisation en tant que médicament.

13. Composition pharmaceutique pour une utilisation selon la revendication 12 dans le traitement de l'asthme, de la BPCO, de la bronchite, de la mucoviscidose, d'un cancer du poumon, d'un diabète ou de migraines.
